# EUROPEAN PATENT APPLICATION

(11) **EP 4 817 430 A1**
(43) Date of publication of application: **07.10.2026**
(21) Application number: 24895184.0
(22) Date of filing: 29.11.2024
(51) Int. Cl.: A23L 27/21, A23L 2/38, A23L 2/52, A23L 5/00, A23L 7/104, A23L 11/50, A23L 27/00, A23L 27/24, A23L 27/50, A23L 27/60, A23L 29/00, A23L 33/175, A23L 33/195, C12G 3/08, C12N 1/14, C12N 15/31, C12P 13/00

(54) **SAVORINESS AND/OR SALTINESS ENHANCER, AND FOOD OR BEVERAGE PRODUCT CONTAINING SAME**

(30) Priority: 29.11.2023 JP 2023201275; 29.11.2023 JP 2023201276; 29.11.2023 JP 2023201278; 29.11.2023 JP 2023201280
(71) Applicant: Kikkoman Corporation, Noda-shi, Chiba 278-8601 (JP)
(72) Inventor: ICHIKAWA, Keiichi, Noda-shi, Chiba 278-8601 (JP); TAJIMA, Ryoko, Noda-shi, Chiba 278-8601 (JP)
(74) Representative: TBK
(86) International application number: PCT/JP2024/042318
(87) International publication number: WO 2025/116009

(57) **Abstract**

Provided is a umami and/or saltiness enhancer capable of enhancing umami and/or saltiness of a food or beverage product. In addition, provided is a food or beverage product, such as a food, beverage, and seasoning, containing the umami and/or saltiness enhancer and having enhanced umami and/or saltiness due to a umami-enhancing effect thereof.

The umami and/or saltiness enhancer for a food or beverage product, contains ergothioneine as an active ingredient, in which a food or beverage product having enhanced umami and/or saltiness is obtained by causing ergothioneine to be contained in an amount of 0.005% by weight or more relative to a content of a single umami component in the food or beverage product.

## Description

### TECHNICAL FIELD

The present invention relates to umami and/or saltiness enhancer that enhances umami and/or saltiness of a food or beverage product, and a food or beverage product such as food, beverage, and seasoning in which umami and/or saltiness is enhanced, which contains the umami and/or saltiness enhancer.

### BACKGROUND ART

In recent years, there has been a growing social demand to reduce the intake of salt (sodium chloride) in order to prevent diseases such as hypertension, renal disease, and heart disease, and there has been a trend toward salt reduction in food or beverage products in general. However, when the salt content is simply reduced from that of a conventional product, the taste becomes flat and unpalatable, and when a salt substitute such as potassium chloride is used, palatability is impaired due to off-flavors such as bitterness. A salt-reduction method is known in which a reduction in saltiness is compensated for by increasing umami (savory taste) derived from broth or the like so as to provide satisfactory palatability; however, this method is limited to food or beverage products in which umami is an important taste attribute, and in such cases, it is necessary to considerably increase the amount of the umami component.

Accordingly, research and development have been conducted on saltiness-enhancing substances capable of enhancing the umami of food or beverage products even when the amount of salt is reduced, and on umami-enhancing substances capable of enhancing the umami of food or beverage products even when the salt content is low.

As such saltiness-enhancing substances, Patent Literature 1 discloses a saltiness enhancer containing methional, 4-hydroxy-3(2H)-furanones, or 3-hydroxy-2(5H)-furanones as an active ingredient, and Patent Literature 2 discloses a saltiness enhancer containing niacin, salicin, or helicin; however, many of these saltiness-enhancing substances are chemically synthesized substances, and some of them have not been confirmed to be safe as foods.

As umami-enhancing substances, Patent Literature 3 discloses an umami enhancer containing N-(1-deoxy-D-fructos-1-yl)-pyroglutamic acid, N-(1-deoxy-D-fructos-1-yl)-valine, or N-(1-deoxy-D-fructos-1-yl)-methionine as an active ingredient, and Patent Literature 4 discloses an umami enhancer containing a pyroglutamyl dipeptide as an active ingredient. Components contributing to the umami are mainly amino acids and nucleic acids, and it has been known that some nucleic acids have umami-enhancing effect when combined with glutamic acid.

In addition, Patent Literature 5 discloses a saltiness and/or umami enhancer containing trehalose as an active ingredient.

The trend toward salt reduction is no exception even for soy sauce, which is indispensable to Japanese cuisine, and technological development has been pursued for producing soy sauce having good saltiness and favorable taste characteristics even when the salt content is low.

For example, Patent Literature 6 discloses a soy sauce in which the umami of glutamic acid is enhanced by containing methional, and Patent Literature 7 reports a soy sauce containing 0.5 to 5% (w/v) sodium chloride and 0.2 to 4% (w/v) ammonium ions and having a pH of 3.2 to 5.0, the soy sauce being excellent in saltiness and soy-sauce-like quality.

In addition, Patent Literature 8 discloses that, by using a saltiness and/or an umami enhancer containing trehalose as an active ingredient to enhance the saltiness and umami of sodium chloride, it is possible to produce a food or beverage product having enriched palatability and excellent taste.

### Prior Art Documents

### [Patent Literatures]

Patent Literature 1: Japanese Patent Application Laid-Open No. 2012-223147
Patent Literature 2: Japanese Patent Application Laid-Open No. 2023-135861
Patent Literature 3: Japanese Patent Application Laid-Open No. 2012-29615
Patent Literature 4: Japanese Patent Application Laid-Open No. 2012-29616
Patent Literature 5: Japanese Patent Application Laid-Open No. H10-66540
Patent Literature 6: Japanese Patent Application Laid-Open No. 2014-18146
Patent Literature 7: Japanese Patent No. 5812864
Patent Literature 8: Japanese Patent Application Laid-Open No. H10-66540
Patent Literature 9: PCT International Publication No. WO2019/240243
Patent Literature 10: PCT International Publication No. WO2023/234307

### [Non-Patent Literature]

Non-Patent Literature 1: BIOSCIENCE BIOTECHNOLOGY AND BIOCHEMISTRY (2019) Vol. 83, No. 1, p. 181-184

### SUMMARY

### Technical Problem

An object of the present invention is to provide an umami and/or saltiness enhancer capable of enhancing umami and/or saltiness of a food or beverage product. Another object of the present invention is to provide a food or beverage product, such as a food, beverage, and seasoning, containing the umami and/or saltiness enhancer and having enhanced umami and/or saltiness due to an umami- and/or saltiness-enhancing effect thereof.

### Solution to Problem

As a result of intensive studies on substances capable of enhancing the umami and/or saltiness, the present inventors have found that ergothioneine, which can be produced only by certain microorganisms such as fungi including mushrooms and koji molds, actinomycetes, and cyanobacteria, is capable of enhancing the umami and/or saltiness of a food or beverage product containing an umami component and/or a saltiness component when contained therein, thereby completing the present invention.

The present invention relates to an umami and/or saltiness enhancer according to [1] below, or a food or beverage product according to [2] to [7] below.
[1] An umami and/or saltiness enhancer comprising ergothioneine as an active ingredient.
[2] A food or beverage product having enhanced umami, containing ergothioneine as an umami enhancer in an amount of 0.005% by weight or more relative to a content of a single umami component in the food or beverage product.
[3] A food or beverage product containing salt and having enhanced saltiness, containing ergothioneine as a saltiness enhancer at any of the following concentrations:
   (1) 0.5 ppm or more of the ergothioneine in a food or beverage product having a salt content of 0.1% by weight or more and less than 5% by weight; or
   (2) 0.1 ppm or more of the ergothioneine in a food or beverage product having a salt content of 5% by weight or more.
[4] The food or beverage product according to [2] or [3], in which the food or beverage product having enhanced umami and/or saltiness is soy sauce, miso, fish sauce, mirin, bonito broth, tsuyu, an umami seasoning, a fermented umami seasoning, ketchup, sauce, or mycoprotein.
[5] The food or beverage product according to any one of [2] to [4], in which the food or beverage product is produced using a high ergothioneine-producing koji mold strain.
[6] A food or beverage product having enhanced umami and/or saltiness, containing 10 ppm or more of ergothioneine.
[7] A food or beverage product having enhanced umami and/or saltiness, containing 10 ppm or more of ergothioneine, which is not a soy sauce for retort food, a retort food containing soy sauce, or a food or beverage product containing an ergothioneine-producing fungal fermentation biomass colored with a pigment or a supernatant thereof.

In addition, the present invention relates to a soy sauce according to [8] and [9] below, a food or beverage product according to [10] and [11] below, an ergothioneine-rich koji mold fermented product according to [12] below, a method for enhancing umami and/or saltiness according to [13] below, or use of ergothioneine according to [14] below.
[8] A soy sauce or a soy sauce-containing seasoning, other than a soy sauce for retort food, containing 10 ppm or more of ergothioneine.
[9] The soy sauce according to [8], in which the soy sauce is a reduced-salt soy sauce.
[10] The food or beverage product according to [5], in which the food or beverage product is rice koji, which is an ergothioneine-rich koji mold fermented product containing 0.3 g or more of ergothioneine per 1 kg of a dry koji mold fermented product, obtained by culturing the high ergothioneine-producing koji mold strain in a medium composed of a raw material having a protein content of 5% or more and less than 10%.
[11] The food or beverage product according to [5], in which the food or beverage product is mycoprotein or soy sauce koji, which is an ergothioneine-rich koji mold fermented product containing 0.4 g or more of ergothioneine per 1 kg of a dry koji mold fermented product, obtained by culturing the high ergothioneine-producing koji mold strain in a medium composed of a raw material having a protein content of 10% or more.
[12] An ergothioneine-rich koji mold fermented product containing 50 to 50,000 ppm of ergothioneine.
[13] A method for enhancing umami and/or saltiness of a food or beverage product, including incorporating ergothioneine into the food or beverage product.
[14] Use of ergothioneine for enhancing umami and/or saltiness of a food or beverage product.

### Advantageous Effects of Invention

According to the umami and/or saltiness enhancer of the present invention, umami and/or saltiness can be brought out by adding a very small amount of ergothioneine, so that the umami and/or saltiness can be naturally enhanced without impairing the flavor. When the enhancer is added to a food or beverage product having umami to enhance the umami, saltiness can be perceived more strongly even when a content of salt in the food or beverage product is reduced; therefore, by eating or drinking a food or beverage product such as a seasoning, food, and beverage, whose umami has been enhanced by adding the enhancer, the palatability is maintained and the salt intake can be easily reduced.

By adding an extremely small amount of ergothioneine to a food or beverage product containing an umami component such as monosodium L-glutamate, sodium inosinate, and sodium guanylate, the umami can be enhanced even in the absence of salt.

Soy sauce containing an extremely small amount of ergothioneine has enhanced saltiness and umami, so that natural deliciousness of soy sauce is brought out without impairing the flavor even when a salt content of the soy sauce is reduced. Therefore, it is particularly effective for a reduced-salt soy sauce, and ergothioneine also enhances sweetness of the soy sauce. In addition, the soy sauce produced using the high ergothioneine-producing koji mold strain has an advantage that not only the taste is improved, but also pressability in a filtration step of soy sauce production is improved, resulting in improved workability.

In addition, the koji mold fermented product produced using the high ergothioneine-producing koji mold strain has a high ergothioneine content, can be used as a food, food material, or food raw material that can be eaten as it is to sufficiently ingest ergothioneine, and due to the high ergothioneine content, it becomes a more delicious food with enhanced taste characteristics such as umami, sweetness, and saltiness. Furthermore, the koji mold fermented product of the present invention has a favorable aroma because it has little odor peculiar to koji mold fermented products, such as koji odor, rice bran odor, musty odor, and oxidized odor.

### DESCRIPTION OF EMBODIMENTS

The present invention relates to an umami and/or saltiness enhancer for a food or beverage product containing ergothioneine as an active ingredient, and to a food or beverage product having enhanced umami and/or saltiness, containing ergothioneine which is an umami and/or saltiness enhancer.

In the present invention, "%" means "% by weight" even when the unit is not explicitly specified, and 1 ppm is 0.0001% by weight.

Ergothioneine (hereinafter also referred to as "ERG") is discovered as a sulfur-containing amino acid isolated from the rye ergot fungus (Claviceps purpurea), and has been confirmed to be present also in vivo in plants and animals. However, plants and animals cannot synthesize the ergothioneine, and ergothioneine in vivo is considered to be derived from ergothioneine synthesized by microorganisms such as basidiomycetes. It is known to be contained in some mushrooms of basidiomycetes, for example, edible mushrooms such as oyster mushrooms, shiitake mushrooms, maitake mushrooms, and king oyster mushrooms, and is particularly abundant in golden oyster mushrooms (Tamogitake). It is also known that koji molds produce the ERG.

Regarding the ERG, a biosynthesis pathway from histidine in microorganisms is known, and a sulfur atom is supplied from cysteine. The ERG has high antioxidant properties, and has been reported to have an elastase inhibitory effect and a tyrosinase inhibitory effect, attracting particular attention in the cosmetic and food fields such as skin whitening and wrinkle prevention. In addition, it has been found that the ERG is involved in the biological antioxidant defense system, and its application in the medical field is also being attempted. The ERG has high thermal stability and pH stability, and retains its antioxidant activity even at high temperatures. In addition to being incorporated into foods for expected physiological effects, the ERG is also expected to be used as an antioxidant in foods.

As methods for producing the ERG, extraction from basidiomycetes such as golden oyster mushrooms, chemical synthesis, and fermentation using microorganisms are carried out. The extraction from basidiomycetes such as golden oyster mushrooms takes time to obtain raw materials and is not suitable for mass production. Chemical synthesis is suitable for mass production, but requires the use of expensive synthesis reagents. Therefore, research has been conducted on fermentation-based production methods, such as fermentation using C1 compound-assimilating bacteria or yeast, fermentation using microorganisms engineered to overexpress ERG biosynthetic genes, and solid culture using koji molds into which ERG biosynthetic genes have been introduced so as to overexpress ERG (Non-Patent Literature 1).

As the umami and/or saltiness enhancer according to the embodiment of the present invention, a commercially available ERG product by chemical synthesis may be used, or an extract from mushrooms containing ERG, a koji mold producing ERG and a culture thereof, or sake lees produced using a koji mold, or a purified product thereof may be used. In addition, for food or beverage products using koji mold as a raw material, the amount of ERG in the food or beverage product can be increased by using a koji mold that highly produces ERG, without adding ERG.

As the mushrooms used for the extraction of ERG, mushrooms belonging to the genus Pleurotus of the family Pleurotaceae, such as golden oyster mushroom (Pleurotus cornucopiae var. citrinopileatus), oyster mushroom (Pleurotus ostreatus), shiitake mushroom (Lentinula edodes), and maitake mushroom (Grifola frondosa), can be preferably mentioned because of their high ERG content, and one or a combination of two or more of these can be used.

As the koji mold that produces ERG, any fungus belonging to the genus Aspergillus can be used. Examples thereof include Aspergillus oryzae, Aspergillus sojae, Aspergillus niger, Aspergillus luchuensis, and Aspergillus tamarii. As the strain of the koji mold, for example, a strain available from a depository institution such as Aspergillus oryzae RIB326 strain, a strain included in a commercially available seed koji, or a strain isolated and obtained from a food or beverage production environment such as a sake or soy sauce brewery can be used.

In addition, as the koji mold that produces ERG, in addition to the above-described wild strains, a mutant strain highly producing ERG can be isolated and used by a general mutagenesis method. Examples of the mutagenesis method include treatment with ultraviolet (UV) or X-ray irradiation that physically damages DNA to introduce mutations, and treatment with alkylating agents such as N-methyl-N'-nitro-N-nitrosoguanidine (NTG) and ethyl methanesulfonate (EMS) that chemically damage DNA to introduce mutations.

Furthermore, a high ERG-producing koji mold strain into which a gene related to ERG biosynthesis is introduced by genetic recombination can be used. Examples thereof include, but are not limited to, a koji mold mutant strain into which the egtA gene has been introduced (see Patent Literature 9 and Non-Patent Literature 1), and a mutant strain of the genus Aspergillus in which ERG production has been increased by introducing the AO090005000664 gene specific to the genus Aspergillus (amino acid sequence encoded thereby: SEQ ID No: 1), or an ortholog gene thereof, into which a specific genetic mutation has been introduced, such as a G428S mutation, a C459F mutation, and a deletion of 1 to 112 amino acids in the region from W404 to Q515 (see Patent Literature 10).

As used herein, the term "high ERG-producing koji mold strain" refers to a strain that produces ERG in an amount 5 to 10 times or more, preferably 20 times or more, which is greater than that produced by a non-mutated or non-transformed strain when cultured in liquid medium at 20°C to 30°C for 3 to 14 days in Puffmin SM medium (a solution prepared by adding 5 to 10% Puffmin SM (manufactured by Kikkoman Corporation) to water and suspending it therein). Examples thereof include an Aspergillus sojae strain in which the above-described egtA gene is forcibly expressed, or an Aspergillus oryzae mutant strain containing an amino acid mutation or amino acid deletion in the region of W404 to Q515 of the above-described mutant AO090005000664 gene and an ortholog gene thereof.

The AO090005000664 gene is cloned from the Aspergillus oryzae RIB40 strain, and has a plurality of conserved regions highly conserved with other species. In particular, a region corresponding to amino acid positions 411 to 470 of SEQ ID No: 1 is mentioned as a conserved region, and by introducing a substitution mutation at a position corresponding to position 428 and/or 459 in this conserved region, the production of ERG in the mutant strain increases by 5 to 10 times or more. Similarly, even when a deletion mutation in which 1 to 112 amino acids in the region corresponding to positions 404 to 515 are deleted is introduced, the production of ERG increases by 5 to 10 times or more.

When the food or beverage product is a product using a koji mold fermented product as a raw material, for example, soy sauce, sake, sweet sake (amazake), mirin, miso, or the like, the ERG content in the food or beverage product can be increased by producing koji using a high ERG-producing koji mold strain, even without adding ERG during production. In particular, when a high ERG-producing koji mold strain is used for producing a fermented food, a fermented beverage, or a fermented seasoning, effects other than umami and/or saltiness enhancement are also exhibited as compared to the case where ERG is added.

The enhancement of umami and/or saltiness means that the umami and/or saltiness provided thereby is perceived more strongly as compared to the case without the umami and/or saltiness enhancer according to the embodiment of the present invention. Specifically, it means that, by adding the umami and/or saltiness enhancer, equivalent umami and/or saltiness can be perceived even when the umami and/or saltiness component is reduced by, for example, 1% to 50%. It also means that, by adding the umami and/or saltiness enhancer, umami and/or saltiness equivalent to that in the case of containing, for example, 1.1 to 2 times the amount of the umami and/or saltiness component can be perceived. Alternatively, it means that, when an umami component and/or saltiness is present in a small amount but cannot be perceived, the umami and/or saltiness becomes perceivable for the first time by addition of the umami and/or saltiness enhancer.

The umami component in the present invention is monosodium L-glutamate, sodium inosinate, or sodium guanylate, and the content of a single umami component in the present invention means the content of any one umami component among these three components contained in the food or beverage product.

In a food or beverage product containing the umami component, by incorporating ergothioneine as an umami enhancer in an amount of 0.005% by weight or more relative to the content of the single umami component, the umami of the food or beverage product is enhanced. Specifically, the umami is enhanced by incorporating 1 ppm or more of monosodium L-glutamate as an umami component in a 2% solution, and 1 ppm or more in a 5% solution of sodium inosinate, sodium guanylate, or the like. In a food or beverage product containing two or more umami components, the ERG may be incorporated in an amount of 0.005% by weight or more relative to the content of the smallest umami component.

In addition, the umami enhancer in the present invention exerts an umami -enhancing effect on general food or beverage products by being incorporated in an amount of 10 ppm or more, for example, 10 ppm or more, 15 ppm or more, 20 ppm or more, or 30 ppm or more, 40 ppm or more, or 50 ppm or more.

Since the umami enhancing effect does not necessarily increase even when a large amount of the umami enhancer in the present invention is incorporated, considering economic efficiency, the ERG can be incorporated into the food or beverage product in an amount of 0.15% by weight or less, for example, 0.12% or 0.09% by weight or less, or 0.06% or 0.03% by weight or less. Relative to the content of the umami component of the food or beverage product, the ERG can be incorporated in an amount of 15% by weight or less, for example, 12% or 9% by weight or less, or 6% or 3% by weight or less. The umami enhancer in the present invention exerts an umami-enhancing effect on a food or beverage product at contents of all combinations of these lower limit values and upper limit values for various umami components in the food or beverage product.

The saltiness component in the present invention is salt (NaCl), and the saltiness enhancer in the present invention exerts a saltiness enhancing effect on general food or beverage products by being incorporated in an amount of 1 ppm or more, for example, 1, 5, 10, 15, 20, or 30 ppm or more, or 40 ppm or 50 ppm or more.

Specifically, in a food or beverage product containing salt, by incorporating ergothioneine as a saltiness enhancer in an amount of 0.5 ppm or more in a food or beverage product having a salt content of 0.1% by weight or more and less than 5% by weight, or in an amount of 0.1 ppm or more in a food or beverage product having a salt content of 5% by weight or more, the saltiness of the food or beverage product is enhanced.

In addition, since the saltiness enhancing effect does not necessarily increase even when a large amount of the saltiness enhancer of the present invention is incorporated, considering economic efficiency, the ERG can be incorporated into the food or beverage product in an amount of 0.15% by weight or less, for example, 0.12% or 0.09% by weight or less, or 0.06% or 0.03% by weight or less. The saltiness enhancer in the present invention exerts a saltiness enhancing effect on a food or beverage product at contents of all combinations of these lower limit values and upper limit values for various saltiness components in the food or beverage product.

The concentration of ERG is measured by LCMS under the following conditions.

### (HPLC conditions)

HPLC analysis is performed under the following conditions.
Apparatus: HPLC apparatus; HPLC: Nexera series set (manufactured by Shimadzu Corporation)
Column: COSMOSIL 2.5HILIC column 3.0 x 150 2.5 µm 3.0 mm I.D. x 15.0 cm (manufactured by Nacalai Tesque, Inc.)
Flow rate: 0.5 mL/min
Temperature: 40°C
Mobile phase: A) 0.1% (v/v) aqueous formic acid solution, B) 0.1% (v/v) formic acid in acetonitrile
Isocratic: 0 to 10 minutes (B: 80%)
Injection volume: 5 µL

### (Mass spectrometry)

Apparatus: LCMS-2020 (manufactured by Shimadzu Corporation)

### (Mass spectrometry (LC-MS) analysis conditions)

Ionization conditions: ESI+
MS conditions: SIM ERG; m/z: 230.1 ([M+H]⁺)
Retention time: around 5 minutes

The food or beverage product according to the embodiment of the present invention is a food, a beverage, or a seasoning.

The food is not limited and may be any food, preferably a food containing the umami component. Examples thereof include retort pouch foods such as curry, stew, and meat sauce; processed vegetable products such as salads and pickles; seafood products such as salmon, tuna, sardine, shrimp, bonito, saury, squid, scallop, crab, and oyster; processed marine and livestock foods such as kamaboko (boiled fish paste), hamburger steak, ham, and sausage; dairy products; cooked rice products such as rice balls, pilaf, fried rice, rice porridge, ochazuke (rice with green tea), oyakodon (chicken-and-egg rice bowl), chukadon (Chinese-style rice bowl), and katsudon (pork cutlet rice bowl); cooked foods such as spring rolls, shumai, gyoza (dumplings), curry, simmered dishes, and deep-fried dishes; mycoprotein; pet food; and medical foods. Examples of the mycoprotein include yeast-derived proteins, filamentous fungus-derived proteins, and fungus-derived proteins such as mushrooms.

The beverage is also not limited, and examples thereof include soft drinks, nutritional drinks, fruit drinks, lactic acid drinks, sports drinks, soups such as egg soup, seaweed soup, Chinese-style soup, consomme soup, and potage soup, clear soups, miso soup, alcoholic beverages such as beer, shochu, and cocktails, and quasi-drug nutritional drinks. The food or beverage includes not only liquid ones but also gel-like and semi-solid ones such as jelly drinks.

The seasoning is also not particularly limited, and examples thereof include soy sauce, reduced-salt soy sauce, mirin, miso, fish sauce, cooking sake, tsuyu, broths such as bonito broth and kelp broth, umami seasonings, fermented umami seasonings, sushi vinegar, tare, sauce, oyster sauce, various other sauces, ketchup, dressings, and mayonnaise.

The umami seasoning is a chemical seasoning obtained by purifying a compound having umami, and specific examples thereof include amino acid-based seasonings and nucleic acid-based seasonings. Examples of the amino acid-based seasoning include glutamic acid and salts thereof (such as sodium glutamate). Examples of the nucleic acid-based seasoning include sodium inosinate and sodium guanylate. The fermented umami seasoning refers to a proteolytic enzyme degradation product of wheat gluten, soybean, or the like.

In addition, in a process of producing the above-described food or beverage product, when the umami enhancer in the present invention is added to the food or beverage product, the timing of addition may be in any step, and the method of addition is not limited as long as the required amount is contained in the final food or beverage product.

As the soy sauce, brewed soy sauce produced by heat-treating a plant raw material such as soybean and wheat, propagating a koji mold therein, and then fermenting and aging the mixture in a salt solution is representative, and a salt concentration thereof is generally 15% to 18% by weight. Other examples of the soy sauce include chemical soy sauce produced by decomposing plant raw materials with acids or enzymes, fish sauce produced by fermenting seafood, and meat sauce produced by fermenting meat; however, the soy sauce in the present invention is preferably a brewed soy sauce using a koji mold.

There are various types of brewed soy sauce depending on differences in production methods, such as the ratio of soybeans to wheat used as raw materials, methods for treating the raw materials, and salt concentration; and examples thereof include koikuchi (dark soy sauce), usukuchi (light soy sauce), tamari, shiro (white soy sauce), and saishikomi (twice-brewed soy sauce). There is also reduced-salt soy sauce in which the salt content of normal soy sauce is reduced by nearly half, and the soy sauce in the present invention also includes the reduced-salt soy sauce.

By incorporating ERG according to the present invention in an amount of 5 ppm or more, for example, 10 ppm, 15 ppm, 25 ppm, 30 ppm or more, or 35 ppm, 40 ppm, 45 ppm, or 50 ppm or more into soy sauce, the saltiness and taste characteristics such as umami of the soy sauce are enhanced. Similarly, in the case of reduced-salt soy sauce, the saltiness and taste characteristics such as umami of the reduced-salt soy sauce are enhanced by incorporating 5 ppm or more, for example, equal to or more than the above-described concentration.

Furthermore, also for a soy sauce-containing seasoning containing soy sauce, the saltiness and taste characteristics such as umami are enhanced by incorporating 5 ppm or more of ERG.

In addition, since the umami and saltiness enhancing effect on the soy sauce does not necessarily increase even when a large amount of ERG is incorporated, considering economic efficiency, the ERG can be incorporated into the soy sauce in an amount of 0.15% by weight or less, for example, 0.12% or 0.09% by weight or less, or 0.06% by weight or less. The soy sauce in the present invention exerts umami and saltiness enhancing effect at ERG contents of all combinations of these lower limit values and upper limit values.

The ERG-rich koji mold fermented product in the present invention is prepared by fermentation in either a conventional solid medium or a conventional liquid medium for culturing a koji mold. As forms of koji used industrially, there are two forms: solid koji obtained by directly growing a koji mold on soybean, wheat, rice, or the like, and liquid koji obtained by growing a koji mold in a liquid medium in which soybean, wheat, rice, or the like is suspended. The koji mold fermented product in the present invention includes solid koji or liquid koji. As fermentation conditions, ordinary conditions used in the present technical field can be used.

The solid medium used for producing the solid koji includes a solid medium composed of a raw material having a protein content of 5% or more and less than 10% in the solid content (protein amount in the food nutritional composition table), and a solid medium composed of a raw material having a protein content containing 10% or more of protein in the solid content (protein amount in the food nutritional composition table). As a raw material for such a solid medium, for example, rice having a protein content of 6%, which is a raw material having a protein content of 5% or more and less than 10%, and beans, fish, meat, algae, and processed products thereof containing 20% or more of protein, which are raw materials having a protein content of 10% or more, can be used. Examples of the processed product containing 20% or more of protein include defatted soybean, fish meal, meat meal, and spirulina powder. The solid medium can be prepared by steaming or boiling the above-described raw materials in boiling water and optionally crushing them.

In addition, examples of a raw material used for the liquid medium used for producing the liquid koji include wheat bran, rice, defatted soybean, corn, okara (soybean curd residue), yeast extract, and dextrin.

The ERG-rich koji mold fermented product after the completion of fermentation can be recovered by solid-liquid separation of the liquid from the medium, concentrated with an ion exchange resin or the like depending on the intended use, and used as it is or after being dried and optionally seasoned, as an ERG-containing food material serving as a raw material for other foods.

In addition, the ERG-rich koji mold fermented product obtained by using specific materials for the solid medium or liquid medium and, optionally, seasoning the product or the like can be used, for example, as a food in the form of mycoprotein or as a food material in the form of rice koji or soy sauce koji. Therefore, the ERG-rich koji mold fermented product in the present invention can be used as foods or an intermediate raw material for foods that use koji mold fermented products as raw materials, such as fermented foods, fermented beverages, and fermented seasonings.

When an ERG-rich koji mold solid fermented product is produced using the high ERG-producing koji mold strain in the present invention, it is possible to produce an ERG-rich koji mold solid fermented product containing ERG in an amount of, for example, 0.3 g or 0.4 g or more per 1 kg of koji mold solid fermented product, preferably 0.5 g or more per 1 kg of koji mold solid fermented product, more preferably 0.6 g or more per 1 kg of koji mold solid fermented product, and still more preferably 0.8 g or more per 1 kg of koji mold solid fermented product.

Rice koji obtained in a medium composed of rice, which is a raw material having a protein content of 5% or more and less than 10%, becomes an ERG-rich koji mold fermented product containing 0.3 g or more of ERG per 1 kg of dry koji mold fermented product, and soy sauce koji obtained in a medium using soybean and wheat as raw materials and having a protein content of 25%, and mycoprotein obtained in a medium composed of a raw material having a protein content of 10% or more become an ERG-rich koji mold fermented product containing 0.4 g or more of ERG per 1 kg of dry koji mold fermented product.

When an ERG-rich koji mold liquid fermented product is produced using the high ERG-producing koji mold strain in the present invention, an ERG-rich koji mold liquid fermented product containing 50 to 50,000 ppm of ERG can be produced. For example, a koji mold fermented product containing 50 ppm or more, 100, 300, 500, or 1,000 ppm or more of ERG, and containing 5% or less, 4%, 3%, 2%, or 1% or less of ERG is obtained.

### Examples

Hereinafter, the present invention will be specifically described in detail with reference to Examples, but the present invention is not limited thereto.

In Examples, when simply indicated as "%", it means "% by weight". In addition, samples at room temperature were used for sensory evaluation.

### [Test 1: Umami enhancing effect on sodium glutamate solution by ERG]

0.2% NaCl was mixed with pure water. Monosodium L-glutamate (hereinafter, also referred to as "MSG") was added thereto to prepare MSG samples having six concentrations of 0%, 0.75%, 1%, 1.25%, 1.5%, and 1.75%; and as a seventh sample, a sample in which purified ergothioneine (purity: 99.5% or more, Tetrahedron) was added to 1% MSG to a final concentration of 60 ppm was prepared. 0.2% NaCl was added for sensory evaluation because the taste would be flat with MSG alone.

The umami was evaluated by a sensory evaluation by an expert panel consisting of five trained umami-sensitive subjects. The panel of five experts was asked to select a sample having an equivalent umami from among the 1st to 6th MSG samples (the concentration was not disclosed to the panel) regarding the umami of the 7th 1% MSG solution containing 60 ppm ERG. After tasting each sample and before tasting the next sample, the mouth was completely rinsed with water at room temperature.

In the sensory evaluation, regarding the 7th sample, 4 out of the 5 panelists evaluated the umami to be equivalent to that of the 1.25% MSG solution, and 1 panelist evaluated it to be equivalent to that of the 1.5% MSG solution. As a result, all the 5 panelists evaluated that the umami was enhanced by the addition of 60 ppm ERG.

### [Test 2: ERG addition amount for enhancing umami of MSG]

### (1) Case of 2% MSG sample (0.2% NaCl added)

In the same manner as in Test 1, samples were prepared by adding ergothioneine to a 2% MSG sample in which 0.2% NaCl was mixed in pure water so that the final concentration was 0.5 ppm, 1 ppm, 3 ppm, 5 ppm, 10 ppm, and 20 ppm (ERG0 to 20 in Table 1 below; hereinafter, "ERGX" means a sample to which X ppm of ERG is added).

**[Table 1]**

| | ERG0 | ERG0.5 | ERG1 | ERG3 | ERG5 | ERG10 | ERG20 |
|---|---|---|---|---|---|---|---|
| MSG | 2% | 2% | 2% | 2% | 2% | 2% | 2% |
| NaCl | 0.2% | 0.2% | 0.2% | 0.2% | 0.2% | 0.2% | 0.2% |
| ERG | 0 ppm | 0.5 ppm | 1 ppm | 3 ppm | 5 ppm | 10 ppm | 20 ppm |

A tasting test was conducted on 2% MSG samples to which the ERG was added at each concentration (the concentration was not disclosed to the panel) by four expert panelists using a 2% MSG sample to which no ergothioneine was added as a control, and umami was evaluated by sensory evaluation. After tasting each sample and before tasting the next sample, the mouth was completely rinsed with water at room temperature.

Pairs combining the control 2% MSG sample and the 2% MSG sample containing ergothioneine at each concentration were presented to the expert panel, and the panel evaluated which of the presented pairs of samples was perceived to have umami by a paired comparison test. The results are shown in Table 2.

### <Evaluation method of paired comparison test>

### Evaluation results of umami of test product compared with control

∘: Umami was enhanced compared to the control.
◊: Umami was similar to that of the control.

The evaluation method of the paired comparison test and the indication of the evaluation results are the same in other tests.

**[Table 2]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG0.5 | ◊ | ◊ | ◊ | ◊ |
| ERG0 | ERG1 | ○ | ○ | ○ | ○ |
| ERG0 | ERG3 | ○ | ○ | ○ | ○ |
| ERG0 | ERG5 | ○ | ○ | ○ | ○ |
| ERG0 | ERG10 | ○ | ○ | ○ | ○ |
| ERG0 | ERG20 | ○ | ○ | ○ | ○ |
| ERG0 | ERG20 | ○ | ○ | ○ | ○ |
| ERG0 | ERG40 | ○ | ○ | ○ | ○ |

Regarding the 2% MSG samples to which 1 ppm or more of ERG was added, all the panelists evaluated that the umami was enhanced. This corresponds to the addition of ERG in an amount of 0.005% by weight or more relative to the content of MSG as an umami component.

### (2) Case of 1% MSG sample (0.2% NaCl added)

Samples (ERG15 to 900) were prepared by adding ergothioneine to a 1% MSG sample, which was a mixture of 0.2% NaCl in pure water, to final concentrations of 15 ppm, 20 ppm, 40 ppm, 60 ppm, 80 ppm, 100 ppm, 400 ppm, and 900 ppm. A drinking test was conducted on 1% MSG samples containing each concentration of ERG by the same expert panel of four as in Test 2, and umami was evaluated by sensory evaluation. Pairs combining 1% MSG samples containing different concentrations of ERG (for example, a pair of ERG 20 ppm and 40 ppm) were presented to the expert panel of four, and the panel evaluated which of the presented pairs of samples was perceived to have umami by a paired comparison test. The results are shown in Table 3.

**[Table 3]**

| | | Panelist | | | |
|---|---|---|---|---|---|
| Control | Test product | A | B | C | D |
| ERG15 | ERG20 | ○ | ○ | ◊ | ○ |
| ERG20 | ERG40 | ○ | ○ | ○ | ○ |
| ERG40 | ERG60 | ◊ | ○ | ○ | ○ |
| ERG60 | ERG80 | ◊ | ◊ | ○ | ◊ |
| ERG80 | ERG100 | ◊ | ◊ | ◊ | ◊ |
| ERG100 | ERG400 | ◊ | ◊ | ◊ | ◊ |
| ERG400 | ERG900 | ◊ | ◊ | ◊ | ◊ |

Regarding the MSG sample to which 80 ppm of ERG was added, three panelists evaluated that the degree of umami was equivalent to that to which 60 ppm of ERG was added. Therefore, it was evaluated that, even when 80 ppm of ERG was added, the degree of umami enhancement remained the same as when 60 ppm was added, confirming that the umami enhancing effect became constant at 60 ppm of ERG.

From the above, regarding the 1% MSG solution, it was evaluated that, when ERG was added up to 60 ppm, the umami was progressively enhanced as the addition amount increased. When it exceeded 60 ppm, the umami-enhancing effect did not differ from that with the addition of 60 ppm.

### (3) Case of 10% MSG sample (no NaCl)

The same test as in (1) of Test 2 was conducted on 10% MSG (no NaCl). Samples were prepared by adding ergothioneine to a 10% MSG sample to final concentrations of 1 ppm, 3 ppm, 5 ppm, 10 ppm, and 20 ppm (ERG0 to 20 in Table 4 below).

Pairs combining the control 10% MSG sample and the 10% MSG sample containing ergothioneine at each concentration were presented to the expert panel of four, and the panel evaluated which of the presented pairs of samples was perceived to have umami by a paired comparison test. The results are shown in Table 5.

**[Table 4]**

| | ERG0 | ERG1 | ERG3 | ERG5 | ERG10 | ERG20 |
|---|---|---|---|---|---|---|
| MSG | 10% | 10% | 10% | 10% | 10% | 10% |
| ERG | 0 ppm | 1 ppm | 3 ppm | 5 ppm | 10 ppm | 20 ppm |

**[Table 5]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG1 | ◊ | ○ | ○ | ○ |
| ERG0 | ERG3 | ○ | ○ | ○ | ○ |
| ERG0 | ERG5 | ○ | ○ | ○ | ○ |
| ERG0 | ERG10 | ○ | ○ | ○ | ○ |
| ERG0 | ERG20 | ○ | ○ | ○ | ○ |

Regarding the 10% MSG samples in which 1 ppm or more of ERG was added to 10% MSG (no NaCl), three panelists evaluated that the umami was enhanced; and regarding the 10% MSG samples in which 3 ppm or more of ERG was added, all the panelists evaluated that the umami was enhanced.

### [Test 3: Umami enhancing effect of inosinate]

### (1) Case of 5% inosinate sample

Samples (ERG0.1 to 20) were prepared by adding ergothioneine to a 5% inosinate sample, which was a mixture of 0.2% NaCl in pure water, to final concentrations of 0.1 ppm, 0.5 ppm, 1 ppm, 3 ppm, 5 ppm, and 20 ppm.

A drinking test by a panel of four experts was conducted on 5% inosinate samples to which ERG was added at each concentration (the concentration was not disclosed to the panel), using a 5% inosinate sample to which no ergothioneine was added as a control, and umami was evaluated by sensory evaluation. After tasting each sample and before tasting the next sample, the mouth was completely rinsed with water at room temperature.

Pairs combining the control 5% inosinate sample and the 5% inosinate sample containing ergothioneine at each concentration were presented to the expert panel, and the panel evaluated which of the presented pairs of samples was perceived to have umami by a paired comparison test. The results are shown in Table 6.

**[Table 6]**

| | | Panelist | | | |
|---|---|---|---|---|---|
| Control | Test product | A | B | C | D |
| ERG0 | ERG0.1 | ◊ | ◊ | ◊ | ◊ |
| ERG0 | ERG0.5 | ○ | ○ | ◊ | ○ |
| ERG0 | ERG1 | ○ | ○ | ○ | ○ |
| ERG0 | ERG3 | ○ | ○ | ○ | ○ |
| ERG0 | ERG5 | ○ | ○ | ○ | ○ |
| ERG0 | ERG20 | ○ | ○ | ○ | ○ |

Regarding the 5% inosinate samples to which 0.5 ppm of ERG was added, three panelists evaluated that the umami was enhanced, and regarding the 5% inosinate samples to which 1 ppm or more of ERG was added, all the panelists evaluated that the umami was enhanced.

### [Test 4: Saltiness enhancing effect on NaCl solution by ERG]

NaCl was added to pure water to prepare NaCl samples having five concentrations of 0.3%, 0.35%, 0.4%, 0.45%, and 0.5%; and as a sixth sample, a sample in which purified ergothioneine (purity: 99.5% or more, Tetrahedron) was added to 0.4% NaCl to a final concentration of 60 ppm was prepared.

The saltiness was evaluated by a sensory evaluation by an expert panel consisting of five trained saltiness-sensitive subjects. The panel of five experts was asked to select a sample having an equivalent saltiness from among the 1st to 5th NaCl samples (the concentration was not disclosed to the panel) regarding the saltiness of the 6th 0.4% NaCl solution containing 60 ppm ERG. After tasting each sample and before tasting the next sample, the mouth was completely rinsed with water at room temperature.

As a result of the sensory evaluation, regarding the 6th sample of 0.4% NaCl containing 60 ppm ERG, all five panelists evaluated the saltiness to be equivalent to that of the 0.45% NaCl solution. As a result, all the five panelists evaluated that the saltiness was enhanced by the addition of 60 ppm ERG.

### [Test 5: ERG addition amount for enhancing saltiness of NaCl]

### (1) Case of 0.1% to 20% NaCl samples

Samples were prepared by adding ergothioneine to 0.1% to 20% NaCl samples to final concentrations of 0.05 ppm, 0.1 ppm, 0.25 ppm, and 0.5 ppm (ERG0.05 to 0.5 in Table 7 below; hereinafter, "ERGX" means a sample to which X ppm of ERG is added).

**[Table 7]**

| | ERG0 | ERG0.05 | ERG0.1 | ERG0.25 | ERG0.5 |
|---|---|---|---|---|---|
| NaCl | 0.1% to 20% | | | | |
| ERG | 0 ppm | 0.05 ppm | 0.1 ppm | 0.25 ppm | 0.5 ppm |

A drinking test by an expert panel of four was conducted on 0.1% to 20% NaCl samples to which ERG was added at each concentration (the concentration was not disclosed to the panel), using 0.1% to 20% NaCl samples to which no ergothioneine was added as a control. Pairs combining the control 0.1% to 20% NaCl sample and the 0.1% to 20% NaCl sample containing ergothioneine at each concentration were presented to the expert panel, and the panel evaluated which of the presented pairs of samples was perceived to have saltiness by a paired comparison test. The results are shown in Table 8.

### <Evaluation method of paired comparison test>

### Evaluation results of saltiness of test product compared with control

∘: Saltiness was enhanced compared to the control.
◇: Saltiness was similar to that of the control.

The evaluation method of the paired comparison test and the indication of the evaluation results are the same in other tests.

**[Table 8]**

| Panelist A | | | | | |
|---|---|---|---|---|---|
| Concentration of NaCl | Control | Test product | | | |
| | ERG0 | ERG0.05 | ERG0.1 | ERG0.25 | ERG0.5 |
| 0.10% | ◊ | ◊ | ◊ | ○ | ○ |
| 0.25% | ◊ | ◊ | ◊ | ○ | ○ |
| 0.50% | ◊ | ◊ | ○ | ○ | ○ |
| 1% | ◊ | ◊ | ○ | ○ | ○ |
| 5% | ◊ | ◊ | ○ | ○ | ○ |
| 10% | ◊ | ◊ | ○ | ○ | ○ |
| 15% | ◊ | ◊ | ○ | ○ | ○ |
| 20% | ◊ | ◊ | ○ | ○ | ○ |

### Panelist B

| Concentration of NaCl | Control | Test product | | | |
|---|---|---|---|---|---|
| | ERG0 | ERG0.05 | ERG0.1 | ERG0.25 | ERG0.5 |
| 0.10% | ◊ | ◊ | ◊ | ◊ | ○ |
| 0.25% | ◊ | ◊ | ○ | ○ | ○ |
| 0.50% | ◊ | ○ | ○ | ○ | ○ |
| 1% | ◊ | ○ | ○ | ○ | ○ |
| 5% | ◊ | ○ | ○ | ○ | ○ |
| 10% | ◊ | ○ | ○ | ○ | ○ |
| 15% | ◊ | ○ | ○ | ○ | ○ |
| 20% | ◊ | ○ | ○ | ○ | ○ |

### Panelist C

| Concentration of NaCl | Control | Test product | | | |
|---|---|---|---|---|---|
| | ERGO | ERG0.05 | ERG0.1 | ERG0.25 | ERG0.5 |
| 0.10% | ◊ | ◊ | ◊ | ○ | ○ |
| 0.25% | ◊ | ◊ | ◊ | ○ | ○ |
| 0.50% | ◊ | ◊ | ○ | ○ | ○ |
| 1% | ◊ | ○ | ○ | ○ | ○ |
| 5% | ◊ | ○ | ○ | ○ | ○ |
| 10% | ◊ | ○ | ○ | ○ | ○ |
| 15% | ◊ | ○ | ○ | ○ | ○ |
| 20% | ◊ | ○ | ○ | ○ | ○ |

### Panelist D

| Concentration of NaCl | Control | Test product | | | |
|---|---|---|---|---|---|
| | ERG0 | ERG0.05 | ERG0.1 | ERG0.25 | ERG0.5 |
| 0.10% | ◊ | ◊ | ◊ | ○ | ○ |
| 0.25% | ◊ | ◊ | ◊ | ○ | ○ |
| 0.50% | ◊ | ◊ | ○ | ○ | ○ |
| 1% | ◊ | ○ | ○ | ○ | ○ |
| 5% | ◊ | ○ | ○ | ○ | ○ |
| 10% | ◊ | ○ | ○ | ○ | ○ |
| 15% | ◊ | ○ | ○ | ○ | ○ |
| 20% | ◊ | ○ | ○ | ○ | ○ |

The results of the sensory evaluation showed that in the NaCl samples of 0.1% or more and less than 0.5%, when 0.5 ppm or more of ERG was added, all evaluated that the saltiness was enhanced. In the NaCl samples of 0.5% or more, when 0.1 ppm or more of ERG was added, all evaluated that the saltiness was enhanced.

### (2) Case of 3% NaCl sample

Samples (ERG0.5 to 100) were prepared by adding ergothioneine to a 3% NaCl sample to final concentrations of 0.5 ppm, 12 ppm, 24 ppm, 60 ppm, 72 ppm, and 100 ppm. A drinking test by a panel of four experts was conducted on 3% NaCl samples to which ERG was added at each concentration, and the saltiness was subjected to a sensory evaluation. Pairs combining 3% NaCl samples containing different concentrations of ERG (for example, a pair of ERG 24 ppm and 60 ppm) were presented to the expert panel of four, and the panel evaluated which of the presented pairs of samples was perceived to have saltiness by a paired comparison test. The results are shown in Table 9.

**[Table 9]**

| | | Panelist | | | |
|---|---|---|---|---|---|
| Control | Test product | A | B | C | D |
| ERG0 | ERG0.5 | ○ | ○ | ○ | ○ |
| ERG0.5 | ERG 12 | ○ | ○ | ○ | ○ |
| ERG12 | ERG24 | ○ | ○ | ○ | ○ |
| ERG24 | ERG60 | ○ | ○ | ○ | ○ |
| ERG60 | ERG72 | ◊ | ◊ | ◊ | ○ |
| ERG72 | ERG100 | ◊ | ◊ | ◊ | ◊ |

Regarding the 3% NaCl samples to which 0.5 ppm or more of ERG was added, all the panelists evaluated that the saltiness was enhanced.

In addition, regarding the 3% NaCl sample to which 72 ppm of ERG was added, three panelists evaluated that the degree of saltiness was equivalent to that to which 60 ppm of ERG was added. Therefore, it was evaluated that, even when 72 ppm of ERG was added, the degree of saltiness enhancement remained the same as when 60 ppm was added, confirming that the saltiness enhancing effect became constant at 60 ppm of ERG.

From the above, regarding the 3% NaCl solution, it was evaluated that, when ERG was added at 0.5 ppm to 60 ppm, the saltiness was progressively enhanced as the addition amount increased. When it exceeded 60 ppm, the saltiness enhancing effect did not differ from that with the addition of 60 ppm.

### [Test 6: ERG addition amount for enhancing umami of soy sauce]

### (1) Case of koikuchi soy sauce

Samples (ERG5 to 200) were prepared by adding ergothioneine to an undiluted koikuchi soy sauce solution to addition concentrations of 5 ppm, 10 ppm, 50 ppm, 100 ppm, and 200 ppm.

A drinking test by a panel of five experts was conducted on koikuchi soy sauce to which ERG was added at each concentration (the concentration was not disclosed to the panel), using a soy sauce to which no ergothioneine was added as a control. Pairs combining the control soy sauce and the soy sauce containing ergothioneine at each concentration were presented to the expert panel, and the panel evaluated which of the presented pairs of samples was perceived to have umami by a paired comparison test. The results are shown in Table 10.

**[Table 10]**

| | | Panelist | | | | |
|---|---|---|---|---|---|---|
| Control | Test product | A | B | C | D | E |
| ERG0 | ERG5 | ○ | ○ | ○ | ◊ | ○ |
| ERG0 | ERG10 | ○ | ○ | ○ | ○ | ○ |
| ERG0 | ERG50 | ○ | ○ | ○ | ○ | ○ |
| ERG0 | ERG100 | ○ | ○ | ○ | ○ | ○ |
| ERG0 | ERG200 | ○ | ○ | ○ | ○ | ○ |

Regarding the soy sauce to which 5 ppm or more of ERG was added, four panelists evaluated that the umami was enhanced, and regarding the soy sauce to which 10 ppm or more of ERG was added, all the panelists evaluated that the umami was enhanced.

Samples (ERG5 to 200) were prepared by adding ergothioneine to koikuchi soy sauce to addition concentrations of 5 ppm, 10 ppm, 50 ppm, 100 ppm, and 200 ppm, and they were each diluted 10-fold with pure water. A drinking test by the same expert panel of five was conducted on the soy sauce to which ERG was added at each concentration, and the umami was subjected to a sensory evaluation. Pairs combining soy sauces containing different concentrations of ERG (for example, a pair of ERG 10 ppm and 50 ppm) were presented to the expert panel of five, and the panel evaluated which of the presented pairs of samples was perceived to have umami by a paired comparison test. The results are shown in Table 11.

**[Table 11]**

| | | Panelist | | | | |
|---|---|---|---|---|---|---|
| Control | Test product | A | B | C | D | E |
| ERG0 | ERG5 | ○ | ○ | ○ | ◊ | ○ |
| ERG5 | ERG10 | ○ | ○ | ○ | ○ | ○ |
| ERG10 | ERG50 | ○ | ○ | ○ | ○ | ○ |
| ERG50 | ERG100 | ○ | ◊ | ○ | ○ | ◊ |
| ERG100 | ERG200 | ○ | ◊ | ◊ | ◊ | ◊ |

Regarding the soy sauce to which 200 ppm of ERG was added, four panelists evaluated that the degree of umami was equivalent to that to which 100 ppm of ERG was added. Therefore, it was evaluated that, even when 200 ppm of ERG was added, the degree of umami enhancement remained the same as when 100 ppm was added.

From the above, regarding koikuchi soy sauce, it was evaluated that, when ERG was added at 5 ppm to 100 ppm, the umami was progressively enhanced as the addition amount increased. When it exceeded 100 ppm, the umami-enhancing effect did not differ from that with the addition of 100 ppm.

### (2) Case of specially selected whole soybean soy sauce, usukuchi soy sauce, or reduced-salt soy sauce

Samples (ERG5 to 200) were prepared by adding ergothioneine to a commercially available specially selected whole soybean soy sauce (Kikkoman Corporation) to addition concentrations of 5 ppm, 10 ppm, 50 ppm, 100 ppm, and 200 ppm, and in the same manner as in (1) described above, a drinking test by an expert panel of five was conducted. Pairs combining the control soy sauce and the soy sauce containing ergothioneine at each concentration were presented to the expert panel, and the panel evaluated which of the presented pairs of samples was perceived to have umami by a paired comparison test.

In addition, similar paired comparison tests were also conducted on a commercially available usukuchi soy sauce and reduced-salt soy sauce.

As a result, regarding any of the specially selected whole soybean soy sauce, usukuchi soy sauce, and reduced-salt soy sauce, to which 5 ppm or more of ERG was added, all the panelists evaluated that the umami was enhanced.

### [Test 7: ERG addition amount for enhancing saltiness of soy sauce]

### (1) Case of specially selected whole soybean soy sauce

Samples (ERG5 to 50) were prepared by adding ergothioneine to a commercially available specially selected whole soybean soy sauce (Kikkoman Corporation) to addition concentrations of 5 ppm, 20 ppm, and 50 ppm.

A drinking test by a panel of four experts was conducted on the specially selected whole soybean soy sauce to which ERG was added at each concentration (the concentration was not disclosed to the panel), using a soy sauce to which no ergothioneine was added as a control. Pairs combining the control soy sauce and the soy sauce containing ergothioneine at each concentration were presented to the expert panel, and the panel evaluated which of the presented pairs of samples was perceived to have saltiness by a paired comparison test. The results are shown in Table 12.

**[Table 12]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG5 | ○ | ○ | ○ | ○ |
| ERG0 | ERG20 | ○ | ○ | ○ | ○ |
| ERG0 | ERG50 | ○ | ○ | ○ | ○ |

Regarding the soy sauce to which 5 ppm or more of ERG was added, all the panelists evaluated that the saltiness was enhanced.

### (2) Case of usukuchi soy sauce

A similar paired comparison test was also conducted on a commercially available usukuchi soy sauce. The results are shown in Table 13.

**[Table 13]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG5 | ○ | ○ | ○ | ○ |
| ERG0 | ERG20 | ○ | ○ | ○ | ○ |
| ERG0 | ERG50 | ○ | ○ | ○ | ○ |

Regarding the soy sauce to which 5 ppm or more of ERG was added, all the panelists evaluated that the saltiness was enhanced.

### (3) Case of reduced-salt soy sauce

A similar paired comparison test was also conducted on a commercially available reduced-salt soy sauce. The results are shown in Table 14.

**[Table 14]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG5 | ○ | ○ | ○ | ○ |
| ERG0 | ERG20 | ○ | ○ | ○ | ○ |
| ERG0 | ERG50 | ○ | ○ | ○ | ○ |

Regarding the soy sauce to which 5 ppm or more of ERG was added, all the panelists evaluated that the saltiness was enhanced.

### [Test 8: ERG addition amount for enhancing umami of seasoning]

### (1) Yeast extract

Samples (ERG0.5 to 10) were prepared by adding ergothioneine to a 1% solution of yeast extract (Springer 0402/20-MG-L) to addition concentrations of 0.5 ppm, 1 ppm, 3 ppm, 5 ppm, and 10 ppm, and how the umami and saltiness of the test products changed compared to a control to which ERG was not added was evaluated by a paired comparison test by an expert panel of three. The results of the sensory evaluation are shown in Tables 15 and 16.

**[Table 15]**

| Umami | | | | |
|---|---|---|---|---|
| | | Panelist | | |
| Control | Test product | A | B | C |
| ERG0 | ERG0 | ◇ | ◇ | ◇ |
| ERG0 | ERG0.5 | ○ | ○ | ○ |
| ERG0 | ERG1 | ○ | ○ | ○ |
| ERG0 | ERG3 | ○ | ○ | ○ |
| ERG0 | ERG5 | ○ | ○ | ○ |
| ERG0 | ERG10 | ○ | ○ | ○ |

**[Table 16]**

| Saltiness | | | | |
|---|---|---|---|---|
| | | Panelist | | |
| Control | Test product | A | B | C |
| ERG0 | ERG0 | ◇ | ◇ | ◇ |
| ERG0 | ERG0.5 | ○ | ○ | ○ |
| ERG0 | ERG1 | ○ | ○ | ○ |
| ERG0 | ERG3 | ○ | ○ | ○ |
| ERG0 | ERG5 | ○ | ○ | ○ |
| ERG0 | ERG10 | ○ | ○ | ○ |

Regarding both the umami and saltiness of the yeast extract, all the panelists evaluated that they were enhanced by adding 0.5 ppm or more of ERG.

### [Test 9: ERG addition amount for enhancing saltiness of yeast extract]

Samples (ERG0.5 to 10) were prepared by adding ergothioneine to a 1% solution of yeast extract (Springer 0402/20-MG-L) to addition concentrations of 0.5 ppm, 1 ppm, 3 ppm, 5 ppm, and 10 ppm, and how the saltiness and umami of the test products changed compared to a control to which ERG was not added was evaluated by a paired comparison test by an expert panel of three. The results of the sensory evaluation are shown in Tables 17 and 18.

**[Table 17]**

| Saltiness | | | | |
|---|---|---|---|---|
| | | Panelist | | |
| Control | Test product | A | B | C |
| ERG0 | ERG0 | ◇ | ◇ | ◇ |
| ERG0 | ERG0.5 | ○ | ○ | ○ |
| ERG0 | ERG1 | ○ | ○ | ○ |
| ERG0 | ERG3 | ○ | ○ | ○ |
| ERG0 | ERG5 | ○ | ○ | ○ |
| ERG0 | ERG10 | ○ | ○ | ○ |

**[Table 18]**

| Umami | | | | |
|---|---|---|---|---|
| | | Panelist | | |
| Control | Test product | A | B | C |
| ERG0 | ERG0 | ◇ | ◇ | ◇ |
| ERG0 | ERG0.5 | ○ | ○ | ○ |
| ERG0 | ERG1 | ○ | ○ | ○ |
| ERG0 | ERG3 | ○ | ○ | ○ |
| ERG0 | ERG5 | ○ | ○ | ○ |
| ERG0 | ERG10 | ○ | ○ | ○ |

Regarding both the saltiness and umami of the yeast extract, all the panelists evaluated that they were enhanced by adding 0.5 ppm or more of ERG.

### [Test 10: Taste enhancing effect on various food or beverage products by ERG (Part 1)]

Regarding the test product obtained by adding ERG to a food or beverage product and a control food or beverage product to which ERG was not added, how the taste (umami, sweetness, bitterness) of the test product changed compared to the control was evaluated by a paired comparison test by an expert panel. The results of the sensory evaluation are shown in Tables 19 to 24.

The "ERGX" of the test product means a test product to which X ppm of ERG was added.

### Sensory evaluation

∘: Taste was enhanced compared to the control.
◇: Taste was similar to that of the control.

### (1) Hon-tsuyu

A sensory test was conducted by adding 5 ppm of ERG to an undiluted solution of Hon-tsuyu (Kikkoman Corporation) and then diluting it 4-fold.

**[Table 19]**

| Umami | | | |
|---|---|---|---|
| | | Panelist | |
| Control | Test product | A | B |
| ERG0 | ERG0 | ◇ | ◇ |
| ERG0 | ERG5 | ○ | ○ |

### Sweetness

| | | Panelist | |
|---|---|---|---|
| Control | Test product | A | B |
| ERG0 | ERG0 | ◇ | ◇ |
| ERG0 | ERG5 | ○ | ○ |

By adding 5 ppm of ERG, the umami and sweetness of the Hon-tsuyu were enhanced.

### (2) Zaru-soba tsuyu

A sensory test was conducted after adding 5 ppm of ERG to Zaru-soba tsuyu (straight tsuyu: Kikkoman Corporation).

**[Table 20]**

| Umami | | | | | |
|---|---|---|---|---|---|
| | | Panelist | | | |
| Control | Test product | A | B | C | D |
| ERG0 | ERG0 | ◊ | ◊ | ◊ | ◊ |
| ERG0 | ERG5 | ○ | ○ | ○ | ○ |

### Sweetness

| | | Panelist | | | |
|---|---|---|---|---|---|
| Control | Test product | A | B | C | D |
| ERG0 | ERG0 | ◊ | ◊ | ◊ | ◊ |
| ERG0 | ERG5 | ○ | ○ | ○ | ○ |

By adding 5 ppm of ERG, the umami and sweetness of the Zaru-soba tsuyu were enhanced.

### (3) Miso

A sensory test was conducted after adding 0.5 ppm of ERG to 18 g of miso (Plus Koji Mutenka Koji Bijin, Marukome Co., Ltd.) / 200 ml of hot water.

**[Table 21]**

| Umami | | | |
|---|---|---|---|
| | | Panelist | |
| Control | Test product | A | B |
| ERG0 | ERG0 | ◇ | ◇ |
| ERG0 | ERG0.5 | ○ | ○ |

### Sweetness

| | | Panelist | |
|---|---|---|---|
| Control | Test product | A | B |
| ERG0 | ERG0 | ◇ | ◇ |
| ERG0 | ERG0.5 | ○ | ○ |

By adding 0.5 ppm of ERG, the umami and sweetness of the miso were enhanced.

### (4) Sauce

A sensory test was conducted after adding 5 ppm of ERG to sauce (Delicious Sauce Tonkatsu, Kikkoman Corporation).

**[Table 22]**

| Umami | | | |
|---|---|---|---|
| | | Panelist | |
| Control | Test product | A | B |
| ERG0 | ERG0 | ◇ | ◇ |
| ERG0 | ERG5 | ○ | ○ |

### Sweetness

| | | Panelist | |
|---|---|---|---|
| Control | Test product | A | B |
| ERG0 | ERG0 | ◇ | ◇ |
| ERG0 | ERG5 | ○ | ○ |

By adding 5 ppm of ERG, the umami and sweetness of the sauce were enhanced.

### (5) Shio-koji (salt koji)

A sensory test was conducted after adding 5 ppm of ERG to shio-koji (Plus Koji Nama Shio-koji, Marukome Co., Ltd.).

**[Table 23]**

| Umami | | | | | |
|---|---|---|---|---|---|
| | | Panelist | | | |
| Control | Test product | A | B | C | D |
| ERG0 | ERG0 | ◊ | ◊ | ◊ | ◊ |
| ERG0 | ERG5 | ○ | ○ | ○ | ○ |

### Sweetness

| | | Panelist | | | |
|---|---|---|---|---|---|
| Control | Test product | A | B | C | D |
| ERG0 | ERG0 | ◊ | ◊ | ◊ | ◊ |
| ERG0 | ERG5 | ○ | ○ | ○ | ○ |

By adding 5 ppm of ERG, the umami, saltiness, and sweetness of the shio-koji were enhanced.

### (6) Tomato ketchup

A sensory test was conducted after adding 5 ppm of ERG to tomato ketchup (Del Monte).

**[Table 24]**

| Umami | | | | | |
|---|---|---|---|---|---|
| | | Panelist | | | |
| Control | Test product | A | B | C | D |
| ERG0 | ERG0 | ◊ | ◊ | ◊ | ◊ |
| ERG0 | ERG5 | ○ | ○ | ○ | ○ |

### Sweetness

| | | Panelist | | | |
|---|---|---|---|---|---|
| Control | Test product | A | B | C | D |
| ERG0 | ERG0 | ◊ | ◊ | ◊ | ◊ |
| ERG0 | ERG5 | ○ | ○ | ○ | ○ |

By adding 5 ppm of ERG, the umami and sweetness of the tomato ketchup were enhanced.

### [Test 11: Taste enhancing effect on various food or beverage products by ERG (Part 2)]

Regarding the test product obtained by adding ERG to a food or beverage product and a control food or beverage product to which ERG was not added, how the saltiness of the test product changed compared to the control was subjected to a sensory evaluation by an expert panel. The results of the sensory evaluation are shown in Tables 25 to 29.

The "ERGX" of the test product means a test product to which X ppm of ERG was added.

### Sensory evaluation

∘: Saltiness was enhanced compared to the control.
◊: Saltiness was similar to that of the control.

### (1) Zaru-soba tsuyu

A sensory test was conducted after adding 5 ppm of ERG to Zaru-soba tsuyu (straight tsuyu: Kikkoman Corporation).

**[Table 25]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG5 | ○ | ○ | ○ | ○ |

By adding 5 ppm of ERG, the saltiness of the Zaru-soba tsuyu was enhanced.

### (2) Miso

A sensory test was conducted after adding 5 ppm of ERG to miso (Plus Koji Mutenka Koji Bijin, Marukome Co., Ltd.).

**[Table 26]**

| | | Panelist | |
|---|---|---|---|
| Control | Test product | A | B |
| ERG0 | ERG0 | ◊ | ◊ |
| ERG0 | ERG5 | ○ | ○ |

By adding 5 ppm of ERG, the saltiness of the miso was enhanced.

### (3) Sauce

A sensory test was conducted after adding 5 ppm of ERG to sauce (Delicious Sauce Tonkatsu, Kikkoman Corporation).

**[Table 27]**

| | | Panelist | |
|---|---|---|---|
| Control | Test product | A | B |
| ERG0 | ERG0 | ◊ | ◊ |
| ERG0 | ERG5 | ○ | ○ |

By adding 5 ppm of ERG, the saltiness of the sauce was enhanced.

### (4) Shio-koji

A sensory test was conducted after adding 5 ppm of ERG to shio-koji (Plus Koji Nama Shio-koji, Marukome Co., Ltd.).

**[Table 28]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG5 | ○ | ○ | ○ | ○ |

By adding 5 ppm of ERG, the saltiness of the shio-koji was enhanced.

### (5) Tomato Ketchup

A sensory test was conducted after adding 5 ppm of ERG to tomato ketchup (Del Monte).

**[Table 29]**

| | | Panelist | | | |
|---|---|---|---|---|---|
| Control | Test product | A | B | C | D |
| ERG0 | ERG0 | ◊ | ◊ | ◊ | ◊ |
| ERG0 | ERG5 | ○ | ○ | ○ | ○ |

By adding 5 ppm of ERG, the saltiness of the tomato ketchup was enhanced.

### [Test 12: Sweetness enhancing effect of soy sauce]

### (1) Specially selected whole soybean soy sauce

Soy sauces were prepared by adding ergothioneine to a commercially available specially selected whole soybean soy sauce (Kikkoman Corporation) to addition concentrations of 10 ppm, 20 ppm, and 50 ppm (EGR10, 20, 50).

A drinking test by a panel of three experts was conducted on the soy sauce to which ERG was added at each concentration (the concentration was not disclosed to the panel), using a soy sauce to which no ergothioneine was added (ERG0) as a control, and the sweetness was sensorially evaluated by a paired comparison test. The results are shown in Table 30.

### <Evaluation method of paired comparison test>

### Evaluation results of sweetness of test product compared with control

∘: Sweetness was enhanced compared to the control.
◇: Sweetness was similar to that of the control.

The evaluation method of the paired comparison test and the indication of the evaluation results are the same in other tests.

**[Table 30]**

| | | Panelist | | |
|---|---|---|---|---|
| Control | Test product | A | B | C |
| ERG0 | ERG0 | ◇ | ◇ | ◇ |
| ERG0 | ERG10 | ○ | ○ | ○ |
| ERG0 | ERG20 | ○ | ○ | ○ |
| ERG0 | ERG50 | ○ | ○ | ○ |

Regarding the soy sauce to which 10 ppm or more of ERG was added, all the panelists evaluated that the sweetness was enhanced.

### (2) Amakuchi soy sauce

The same test as in (1) described above was conducted by adding ERG to a commercially available amakuchi soy sauce (Golden Murasaki, Fundokin Shoyu). The results are shown in Table 31.

**[Table 31]**

| | | Panelist | | |
|---|---|---|---|---|
| Control | Test product | A | B | C |
| ERG0 | ERG0 | ◇ | ◇ | ◇ |
| ERG0 | ERG10 | ○ | ○ | ○ |
| ERG0 | ERG20 | ○ | ○ | ○ |
| ERG0 | ERG50 | ○ | ○ | ○ |

Regarding the amakuchi soy sauce to which 10 ppm or more of ERG was added, all the panelists evaluated that the sweetness was enhanced.

### [Test 13: Production of soy sauce]

Steam-denatured defatted soybeans and crushed roasted wheat were mixed in equal amounts, a seed koji was inoculated thereto, and koji-making was carried out for 42 hours by an ordinary method to obtain soy sauce koji. As the seed koji, an Aspergillus sojae mutant strain (Example 1) in which 4 copies of the egtA gene were introduced and the inserted gene was overexpressed, and an Aspergillus oryzae strain (Example 2) in which a G428S mutation was introduced into the AO090005000664 gene were respectively used. 100 parts by mass of the obtained soy sauce koji was charged into 130 parts by mass of a saline solution (salt concentration: 26% (w/v)), and fermented and aged at 25°C to 30°C for 150 days while appropriately stirring, following moromi management according to an ordinary method. The obtained moromi after aging was pressed and filtered to obtain a raw soy sauce. The obtained raw soy sauce was pasteurized at 80°C for 1 hour, and then clarified and filtered to obtain soy sauce. The concentration of ERG in the soy sauce obtained by the clarification and filtration was 566 ppm in Example 1 and 250 ppm in Example 2.

The soy sauce of the Examples had enhanced umami, saltiness, and sweetness compared to commercially available products. Furthermore, the soy sauce of Example 2 had improved pressability in the filtration step during its production, resulting in improved workability.

### [Test 14: Production of mirin]

After raw material processing such as washing with water, soaking in water, draining, steaming, and allowing to cool was performed on polished rice by an ordinary method, 0.1% of seed koji by weight of rice was inoculated and mixed thereto, and koji-making was managed in a thermo-hygrostat at an optimum koji-making temperature of 32°C to 38°C to obtain koji. As the seed koji, an Aspergillus oryzae strain (Example) in which a G428S mutation was introduced into the AO090005000664 ortholog gene and a wild strain thereof (Comparative Example) were respectively used. According to an ordinary method for producing mirin, 48 g of the above-described rice koji (approximately 40 g as raw polished non-glutinous rice) was placed in a preparation container, and mixed with 290 g of glutinous rice (kakemai) that had been washed, soaked in water, drained, steamed, and allowed to cool by an ordinary method. Next, 155 ml of neutral alcohol (alcohol content: 35% (v/v)) was added, and saccharification was carried out at 30°C for 1 month to obtain an aged mirin moromi. Subsequently, the aged mirin moromi was pressed by an ordinary method to obtain a crude mirin filtrate. The crude mirin filtrate was heat-sterilized at a reaching temperature of 90°C, and then clarified and filtered to obtain mirin.

The concentration of ERG in the mirin obtained by the clarification and filtration was 11 ppm in the Example and 2 ppm in the Comparative Example.

The mirin of Example had enhanced not only umami but also sweetness compared to the mirin of Comparative Example.

### [Test 15: Production of mycoprotein]

Fermentation was performed in a 30 L jar (manufactured by Marubishi Bioengineering Co., Ltd.). Water was added to 1.5 kg of okara powder to make up 15 L, and 2.25 g of Shin-Etsu Silicone (registered trademark) KM72F (manufactured by Shin-Etsu Chemical Co., Ltd.) as an antifoaming agent was added, followed by sterilization at 123°C for 60 minutes. An enzyme solution filtered through a filter having a pore size of 0.22 µm was added so that the final concentration of arabinase was 0.6 U/ml and the final concentration of cellulase was 0.1 U/ml, and the mixture was reacted with stirring at 60°C for 2 hours. After the reaction, the mixture was cooled to 30°C.

Pre-culture was performed as follows. For each strain, 5 g of okara powder and 100 ml of water were added to one 500 ml baffled Erlenmeyer flask, and autoclaved (121°C, 30 minutes). As the seed koji, an Aspergillus oryzae strain (Example 1) in which a G428S genetic mutation was introduced into the AO090005000664 ortholog gene, and an Aspergillus oryzae strain (Comparative Example 1) in which the AO090005000664 ortholog gene was wild-type were respectively used. 0.5 to 1 ml of a glycerol stock of the inoculum was added, and inoculated so that the initial number of spores in the pre-culture liquid became 5 x 10⁵/ml. The pre-culture liquid was cultured with shaking for 24 hours (30°C, 160 rpm). Thereafter, the pre-culture liquid was inoculated into the 30 L jar and cultured at 30°C for 3 days. The aeration rate and the agitation speed were initially set at 0.5 vvm and 250 rpm, respectively, and were increased to 1 vvm and 330 rpm after 18 hours. After culturing, the fermented product was subjected to a heat sterilization treatment (80°C, 30 minutes).

The fermented product subjected to the heat sterilization treatment described above was dried for 1 minute with a drum dryer (manufactured by Katsuragi Industry Co., Ltd., product name "Drum Dryer D-00") set at a gap of 0.3 mm and a surface temperature of 150°C. This is referred to as fermented okara. The content of ergothioneine was 1.70 g/kg of dry koji mold fermented product (Example 1) and 0.1 g/kg of dry koji mold fermented product (Comparative Example 1).

Using the fermented okara obtained in the above-described procedure, using 16.7 g of the dried product, the dried product and water were mixed so as to have a ratio of 1:2 on a weight basis, further, 3% of soy sauce and 1% of yeast extract were mixed, and kneaded for 10 seconds with a kneading machine (manufactured by Tiger Corporation, product name "Micom Food Processor SKF-H101"). Thereafter, the kneaded sample was formed into a size of 1.8 cm in thickness and 7.5 cm in inner diameter. 5 ml of oil was put in a frying pan, and the formed sample was baked over low heat for 1 minute and 20 seconds on each side, for a total of 2 minutes and 40 seconds. The content of ergothioneine was 1 g/kg of dry koji mold fermented product (Example 1) and 0.05 g/kg of dry koji mold fermented product (Comparative Example 1).

Regarding the taste of the mycoproteins of Comparative Example 1 and Example 1, a sensory evaluation by an expert panel of five was performed, and all the panelists evaluated that the mycoprotein of Example 1 had stronger umami and was more delicious. In addition, Example 1 is suitable for processing because its off-flavor is weaker than that of Comparative Example 1.

### [Test 16: Production of shio-koji]

### Production of rice koji

1,250 g of white rice was soaked in water overnight, transferred to a colander, and drained for 2 hours. Thereafter, the rice was wrapped in a Tetoron (registered trademark) cloth (Toray Industries, Inc.) and steamed at 100°C for 40 minutes. The steamed rice was cooled to 45°C, and a seed koji was inoculated. As the seed koji, an Aspergillus oryzae strain (Example 2) in which a G428S genetic mutation was introduced into the AO090005000664 ortholog gene, and an Aspergillus oryzae strain (Comparative Example 2) in which the AO090005000664 ortholog gene was wild-type were respectively used. The rice was wrapped in a Pylen cloth, and koji-making was started under the conditions of 35°C and 95% humidity. 19 hours after the start of koji-making, the rice koji was loosened and wrapped again in the Pylen cloth. When the product temperature reached 40°C, the Pylen cloth was spread and the rice koji was leveled. After another 40 hours, the rice koji was well loosened and left to stand again. After 42 hours, the koji was taken out. The content of ergothioneine was 0.35 g/kg of dry koji mold fermented product (Example 2) and 0.03 g/kg of dry koji mold fermented product (Comparative Example 2).

### Production of shio-koji

20 g of salt and 100 ml of hot water at 50°C were added to 50 g of the obtained rice koji, and a reaction was carried out at 50°C and 130 rpm for 14 hours. The content of ergothioneine was 0.1 g/kg of dry koji mold fermented product (Example 2) and 0.01 g/kg of dry koji mold fermented product (Comparative Example 2).

Regarding the taste of the shio-koji of Comparative Example 2 and Example 2, a sensory evaluation by an expert panel of four was performed, and all the panelists evaluated that the shio-koji of Example 2 had stronger umami and was more delicious.

### [Test 17: Production of amazake]

### Production of amazake

To 800 g of the rice koji obtained in Comparative Example 2 and Example 2, a 2.5-fold amount of hot water at 60°C was added, and a saccharification reaction was carried out at 55°C and 200 rpm. After 16 hours, the Brix was adjusted to 18%, and the mixture was transferred to a heat-resistant container and sterilized at 85°C for 30 minutes. The ERG content of the obtained amazake was 115 ppm (Example 3) and 7 ppm (Comparative Example 3). Compared with Comparative Example 3, Example 3 had stronger sweetness, a whiter color, and a stronger chestnut-like aroma, had a better aroma due to less koji odor and rice bran odor, and had a smoother mouthfeel because it contained fewer large particles.

### [Test 18: Production of soy sauce-pickled rice koji]

### Production of soy sauce-pickled rice koji

To 50 g of the rice koji obtained in Comparative Example 2 and Example 2, 4 g of salt and 75 ml of soy sauce were added, and a reaction was carried out at 50°C and 130 rpm for 14 hours. The content of ergothioneine was 0.14 g/kg of dry soy sauce-pickled rice koji (Example 4) and 0.01 g/kg of dry soy sauce-pickled rice koji (Comparative Example 4).

Regarding the taste of the soy sauce-pickled rice koji of Comparative Example 4 and Example 4, a sensory evaluation by an expert panel of four was performed, and all the panelists evaluated that the soy sauce-pickled rice koji of Example 4 had stronger umami and was more delicious.

### [Test 19: Production of Hishio]

### Production method of Hishio

Steam-denatured defatted soybeans and crushed roasted wheat were mixed in equal amounts, a seed koji was inoculated thereto, and koji-making was carried out for 42 hours by an ordinary method to obtain soy sauce koji. As the seed koji, an Aspergillus oryzae strain (Example 5) in which a G428S mutation was introduced into the AO090005000664 ortholog gene, and an Aspergillus oryzae strain (Comparative Example 5) in which the AO090005000664 ortholog gene was wild-type were respectively used. 100 parts by mass of the obtained soy sauce koji was charged into 130 parts by mass of a saline solution (salt concentration: 26% (w/v)), and fermented and aged at 25°C to 30°C for 150 days while appropriately stirring, following moromi management according to an ordinary method. The content of ergothioneine was 0.23 g/kg of Hishio (Example 5) and 0.04 g/kg of Hishio (Comparative Example 5).

Regarding the taste of the Hishio of Comparative Example 5 and Example 5, a sensory evaluation by an expert panel of four was performed, and all the panelists evaluated that the Hishio of Example 5 had stronger umami and was more delicious. In addition, Example 5 had reduced musty odor and oxidized odor compared to Comparative Example 5.

### [Test 20: Production of koji mold fermented seasoning liquid]

### Production method of koji mold fermented seasoning liquid

An Aspergillus oryzae strain in which a G428S mutation was introduced into the AO090005000664 ortholog gene was inoculated into a 2.15% dextrin, 4.5% yeast extract medium, and liquid-cultured at 26°C for 7 days. The obtained culture liquid was heated in a tank at 90°C for 30 minutes, and then the culture liquid was filtered. ERG in the obtained filtrate was adsorbed onto an ion exchange resin UBK08 (manufactured by Mitsubishi Chemical Corporation), and eluted with aqueous ammonia to obtain a koji mold fermented seasoning liquid. The ERG concentration of the obtained koji mold fermented seasoning liquid was 30,000 ppm.

For the koji mold fermented seasoning liquid, enhancing effects on saltiness, sweetness, and umami were observed.

### [SEQUENCE LISTING]

PCT24358KI.xml

## Claims

1. An umami and/or saltiness enhancer comprising ergothioneine as an active ingredient.

2. A food or beverage product having enhanced umami, comprising ergothioneine as an umami enhancer in an amount of 0.005% by weight or more relative to a content of a single umami component in the food or beverage product.

3. A food or beverage product having enhanced saltiness, comprising ergothioneine as a saltiness enhancer at either of the following concentrations in a food or beverage product containing salt:
(1) 0.5 ppm or more of the ergothioneine in a food or beverage product having a salt content of 0.1% by weight or more and less than 5% by weight; or
(2) 0.1 ppm or more of the ergothioneine in a food or beverage product having a salt content of 5% by weight or more.

4. The food or beverage product according to Claim 2 or 3, wherein the food or beverage product having enhanced umami and/or saltiness is soy sauce, miso, fish sauce, mirin, bonito broth, tsuyu, an umami seasoning, a fermented umami seasoning, ketchup, sauce, or mycoprotein.

5. The food or beverage product according to Claim 2 or 3, wherein the food or beverage product is produced using a high ergothioneine-producing koji mold strain.

6. A food or beverage product having enhanced umami and/or saltiness, comprising 10 ppm or more of ergothioneine.

7. A soy sauce or a soy sauce-containing seasoning, other than a soy sauce for retort food, comprising 10 ppm or more of ergothioneine.

8. The soy sauce according to Claim 7, wherein the soy sauce is a reduced-salt soy sauce.

9. The food or beverage product according to Claim 5, wherein the food or beverage product is rice koji, which is an ergothioneine-rich koji mold fermented product containing 0.3 g or more of ergothioneine per 1 kg of a dry koji mold fermented product, obtained by culturing the high ergothioneine-producing koji mold strain in a medium composed of a raw material having a protein content of 5% or more and less than 10%.

10. The food or beverage product according to Claim 5, wherein the food or beverage product is mycoprotein or soy sauce koji, which is an ergothioneine-rich koji mold fermented product containing 0.4 g or more of ergothioneine per 1 kg of a dry koji mold fermented product, obtained by culturing the high ergothioneine-producing koji mold strain in a medium composed of a raw material having a protein content of 10% or more.

11. An ergothioneine-rich koji mold fermented product comprising 50 to 50,000 ppm of ergothioneine.

12. A method for enhancing umami and/or saltiness of a food or beverage product, comprising incorporating ergothioneine into the food or beverage product.
